(19) Europäisches Patentamt
European Patent Office
Office européen des brevets

(11) Veröffentlichungsnummer : **0 011 719**
**B1**

(12) # EUROPÄISCHE PATENTSCHRIFT

(45) Veröffentlichungstag der Patentschrift : **30.09.81**

(21) Anmeldenummer : **79104198.1**

(22) Anmeldetag : **29.10.79**

(51) Int. Cl.³ : **C 07 D403/10**, C 07 D405/04, C 07 D405/14, D 06 L 3/12// C07D249/08

(54) **6-(2-Hydroxy-4-(1,2,3-triazolyl-(2))-phenyl)-2,4-dioxo-1,3,5-trimethyl-hexahydro-s-triazine sowie deren Herstellung und Verwendung als Zwischenprodukte für die Herstellung optischer Aufheller.**

(30) Priorität : **09.11.78 DE 2848670**

(43) Veröffentlichungstag der Anmeldung : **11.06.80 (Patentblatt 80/12)**

(45) Bekanntmachung des Hinweises auf die Patenterteilung : **30.09.81 Patentblatt 81/39**

(84) Benannte Vertragsstaaten : **CH DE FR GB IT**

(56) Entgegenhaltungen : **DE - A - 2 355 116**

(73) Patentinhaber : **BAYER AG**
**Zentralbereich Patente, Marken und Lizenzen**
**D-5090 Leverkusen 1, Bayerwerk (DE)**

(72) Erfinder : **Seng, Florin, Dr.**
**Am Katerbach 46**
**D-5060 Bergisch-Gladbach 2 (DE)**
Erfinder : **Dorlars, Alfons, Dr.**
**Mozartstrasse 32**
**D-5090 Leverkusen 1 (DE)**
Erfinder : **Schellhammer, Carl-Wolfgang, Dr.**
**Katharinenthal 26**
**D-5060 Bergisch-Gladbach 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 011 719 B1

6-[2-Hydroxy-4-<1,2,3-triazolyl-(2)>-phenyl]-2,4-dioxo-1,3,5-trimethyl-hexahydro-s-triazine sowie deren Herstellung und Verwendung als Zwischenprodukte für die Herstellung optischer Aufheller

Gegenstand der Erfindung sind Triazolylphenyl-triazine der Formel

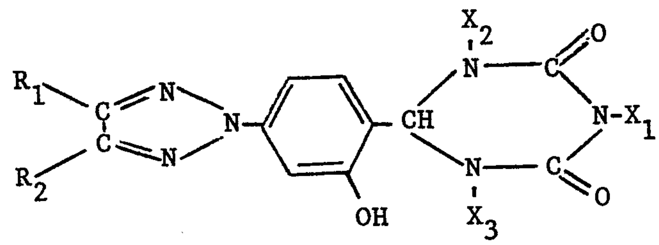

(I)

worin

$R_1$ und $R_2$ $C_1$-$C_4$-Alkyl oder gegebenenfalls durch Halogen, $C_1$-$C_4$-Alkyl oder $C_1$-$C_4$-Alkoxy substituiertes Phenyl

$X_1$, $X_2$ und $X_3$ $C_1$-$C_4$-Alkyl oder Phenyl-$C_1$-$C_4$-Alkyl, wobei der Phenylrest gegebenenfalls durch Halogen, $C_1$-$C_4$-Alkyl oder $C_1$-$C_4$-Alkoxy substituiert ist, bedeuten.

Vorzugsweise stehen $R_1$/$R_2$ für Methyl, Ethyl oder Phenyl und $X_1$-$X_3$ für Methyl.

Unter « Halogen » wird im Rahmen dieser Erfindung vor allem F, Br und insbesondere Cl verstanden.

Die Verbindungen der Formel I werden erfindungsgemäß dadurch hergestellt, daß man 3-[1,2,3-Triazolyl-(2)]-phenole der Formel II

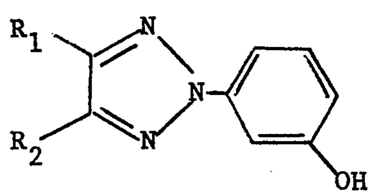

(II)

worin

$R_1$ und $R_2$ die oben angegebene Bedeutung besitzen, mit Verbindungen der Formel

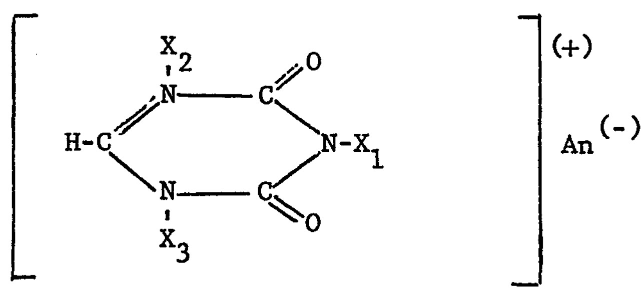

(III)

worin

$X_1$-$X_3$ $An^{(-)}$ die obengenannte Bedeutung besitzen und für ein Anion, vorzugsweise $Cl^{(-)}$ oder $Br^{(-)}$ steht, in Gegenwart eines Säureakzeptors umsetzt.

Die Phenole der Formel II sind bekannt (vgl. Liebigs Annalen der Chemie 1978, S. 345 ff) bzw. in an sich bekannter Weise zugänglich, beispielsweise durch Diazotieren der entsprechenden Anilinderivate und Verkochen der dabei entstehenden Diazoniumsalze.

Auch die Triaziniumsalze der Formel III sind bekannt (vgl. Synthesis 1977, S. 753) und beispielsweise detailiert in der Britischen Patentschrift 1 477 937, Seite 2, Zeilen 43-55, offenbart.

Geeignete Säureakzeptoren sind vor allem organische Basen insbesondere tertiäre Amine, wie z.B. Triäthylamin, Tri-n-propylamin, Tri-n-butylamin und Dimethylbenzylamin.

Diese Basen werden in mindestens 1-molaren Mengen (bezogen auf (III)) eingesetzt.

Die Umsetzung von (II) mit (III) erfolgt vorteilhafterweise in einem polaren organischen Lösungsmittel bei Temperaturen von 80-140 °C, vorzugsweise 90-110 °C, wobei die Molverhältnisse von II/III 1:1 bis 1:1,5 betragen sollen.

Geeignete Lösungsmittel sind Dimethylformamid, Tetramethylharnstoff, Dimethylsulfoxid u.a. Die Reaktionszeiten betragen 1-30 Stunden.

Die erfindungsgemäßen Verbindungen der Formel (I) sind wertvolle Zwischenprodukte zur Herstellung von 4-(1,2,3-Triazolyl-2-)-salicylaldehyden, die ihrerseits Ausgangsmaterialien zur Herstellung

von optischen Aufhellern vom Typ der Triazolylcumarine (vgl. US-PS 4 005 098, US-PS 3 496 188, US-PS 3 646 052 und GB-PS 1 313 253) darstellen.

Gegenüber den bekannten Verfahren zur Herstellung jener Cumarinverbindungen, die von 4-(N-Acylamino)-salicyl-aldehyden ausgehen, zeichnet sich das neue Verfahren unter Verwendung der Verbindungen der Formel (I) als Ausgangsstoffe durch eine geringere Zahl von Reaktionsschritten und damit durch eine verbesserte Raum/Zeit-Ausbeute aus.

Die Spaltung der Verbindungen der Formel (I) zu den entsprechenden freien Aldehyden erfolgt in an sich bekannter Weise durch alkalische Hydrolyse (vgl. GB-PS 1 477 937) und anschließende saure Aufarbeitung.

Bei der praktischen Durchführung der Hydrolyse arbeitet man bei Temperaturen von 90-150 °C in Wasser oder einem mit Wasser mischbaren organischen Lösungsmittel (z.B. n-Butanol oder Methylglykol) in Gegenwart von 5-20 Mol Alkali (z.B. NaOH oder KOH) pro Mol der Verbindung (I).

Man erhält dabei zunächst die Alkalisalze der Aldehyde, die durch Mineralsäuren, wie Salzsäure oder Schwefelsäure, in die freien Salicylaldehyde überführt werden.

Die Umsetzung dieser Aldehyde mit Aryl- oder Heterylessigsäuren in Essigsäureanhydrid in Gegenwart von Natriumacetat nach Perkin ergibt schließlich die entsprechenden Cumarin-Aufheller.

Geeignete (Het)-Arylessigsäuren zur Durchführung dieser Umsetzung sind Phenylessigsäure sowie deren Halogen-, $C_1$-$C_4$-Alkyl- und/oder $C_1$-$C_4$-Alkoxyderivate, Pyrazolylessigsäure, 1,2,3-Triazolylessigsäure-1, 1,2,4-Triazolylessigsäure sowie deren Halogen- oder $C_1$-$C_4$-Alkylderivate.

## Beispiel 1

6-[2-Hydroxy-4-(4-methyl-5-phenyl-1,2,3-triazolyl-(2))-phenyl]-2,4-dioxo-1,3,5-trimethyl-hexahydro-s-triazin

Eine Mischung aus 268 g (1,4 Mol) 2,4-Dioxo-1,3,5-trimethyl-tetrahydro-s-triaziniumchlorid, 251 g (1 Mol) 2-[3-Hydroxy-phenyl]-4-phenyl-5-methyl-y-triazol und 189 g (1,4 Mol) Dimethylbenzylamin in 400 ml Dimethylformamid wird 11 Std. bei 100 °C gerührt. Anschließend dampft man im Wasserstrahlvakuum bei einer Badtemperatur von 90 °C 380 ml Dimethylformamid ab und versetzt den Rückstand mit 500 ml Wasser. Dabei scheiden sich 390 g Rohmaterial mit einem Reinheitsgrad von 82,9 % ab, was einer Ausbeute von 79,5 % entspricht. Schmelzpunkt nach dem Umlösen aus Essigsäure Fp. : 252 °C.

Analyse $C_{21}H_{22}N_6O_3$ (406)
Ber.: C 62,0 % H 5,3 % N 20,7 %
Gef.: C 62,3 % H 5,1 % N 20,7 %

Verwendet man anstelle von 2-[3-Hydroxyphenyl]-4-phenyl-5-methyl-v-triazol das 2 [3-Hydroxyphenyl]-4-äthyl-5-methyl-v-triazol in obiger Umsetzung, so erhält man das entsprechende 6-[2-Hydroxy-4-(4-methyl-5-äthyl-1,2,3-triazolyl-(2))phenyl] 2,4-dioxo-1,3,5-trimethyl-hexahydro-s-triazin vom Fp. 196° (aus Methanol).

## Beispiel 2

4-[4-Methyl-5-phenyl-1,2,3-triazolyl-(2)]-salicylaldehyd

Eine Mischung aus 570 g 6-[2-Hydroxy-4-(4-methyl-5-phenyl-1,2,3-Triazolyl-(2))-phenyl]-2,4-dioxo-1,3,5-trimethyl-hexahydro-s-triazin, 2 940 ml Wasser und 560 g Natriumhydroxyd wird 22 Std. auf 102 °C erhitzt. Man kühlt dann auf 15 °C ab, saugt das abgeschiedene Natriumsalz ab und trägt es in eine Mischung aus 700 g Eis, 700 ml Wasser und 237 ml 37 %ige Salzsäure ein. Nach vierstündigem Rühren wird der entstandene Salicylaldehyd abgesaugt, mit Eiswasser gewaschen und bei 80 °C getrocknet. Man erhält so 353 g Produkt vom F. 121-123 °C, das laut Analyse 98,6 %ig ist. Die Ausbeute beträgt 89 % d.Th.

Das Produkt ist rein genug für weitere Umsetzungen zu 7-Triazolylcumarinen.

## Beispiel 3

4-[4-Methyl-5-äthyl-1,2,4-triazolyl-(2)]-salicylaldehyd

In eine Lösung von 74,2 g Natriumhydroxyd in 930 ml Wasser gibt man bei 40 °C 95 g 6-[2-Hydroxy-4-(4-methyl-5-äthyl-1,2,3-triazolyl-(2))-phenyl]-2,4-dioxo-1,3,5-trimethyl-hexahydro-s-triazin ; die Mischung wird unter Stickstoff 18 Std. rückfließend gekocht. Man trägt den Inhalt des Reaktionsgefäßes dann auf 1 kg Eis und 170 ml 37 %ige Salzsäure aus und rührt 4 Stunden. Das abgeschiedene Produkt wird abgesaugt, mit Eiswasser gewaschen und bei 50 °C getrocknet. Man isoliert 59 g (80 % d.Th.) des obengenannten Salicylaldehyds vom F. 86-88 °C. Die Verbindung ist lt. Analyse 83,1 %ig.

Das Produkt ist rein genug für weitere Umsetzungen zu 7-Triazolylcumarinen.

## Beispiel 4

7-[4-Methyl-5-phenyl-1,2,3-triazolyl-(2)]-3-[4-chlorpyrazolyl-(1)]-cumarin

In 255 g (2,5 Mol) Essigsäureanhydrid werden unter Rühren nacheinander 49,2 g (0,6 Mol) wasserfreies Natriumacetat, 96,4 g (0,6 Mol) 4-Chlorpyrazolyl-(1)-essigsäure und 139,7 g (0,5 Mol) 4-[4-Methyl-5-phenyl-1,2,3-triazolyl-(2)]-salicylaldehyd gemäß Beispiel 2 eingetragen. Die Mischung wird während 18 Stunden zum Rückflußsieden erhitzt. Man kühlt dann auf 100 °C ab und gibt unter weiterer Kühlung 96 g (3 Mol) Methanol hinzu. Man kühlt auf + 10 °C ab und saugt das abgeschiedene Produkt ab. Nach dem Waschen mit Eisessig, Methanol und warmen Wasser erhält man nach dem Trocknen bei 100 °C 157,2 g Rohmaterial, das aus 2 kg 1,2-Dichlorbenzol umgelöst wird. Die Ausbeute an dem obengenannten Cumarin beträgt 131,2 g.

Beispiel 5

7-[4-Methyl-5-phenyl-1,2,3-triazolyl-(2)]-3-phenylcumarin

Wenn man an Stelle des in Beispiel eingesetzten 4-Chlorpyrazolyl-(1)-essigsäure 81,7 g (0,6 Mol) Phenylessigsäure verwendet, isoliert man als Reaktionsprodukt 127,1 g des vorstehend genannten 3-Phenylcumarins.

Beispiel 6

7-[4-Methyl-5-äthyl-1,2,3-triazolyl-(2)]-3-[1,2,4-triazolyl-(1)]-cumarin

Eine Mischung aus 23,1 g (0,1 Mol) 4-[4-Methyl-5-äthyl-1,2,3-triazolyl-(2)]-salicylaldehyd, 15,3 g (0,12 Mol) 1,2,4-Triazolyl-(1)-essigsäure, 9,8 g (0,12 Mol) wasserfreiem Natriumacetat und 51 g (0,5 Mol) Essigsäureanhydrid wird 12 Std. rückfließend gekocht. Nach der wie oben durchgeführten Aufarbeitung erhält man 22,6 g 7-[4-Methyl-5-äthyl-1,2,3-triazolyl-(2)]-3-[1,2,4-triazolyl-(1)]-cumarin.

**Ansprüche**

1. Triazolylphenyltriazine der Formel

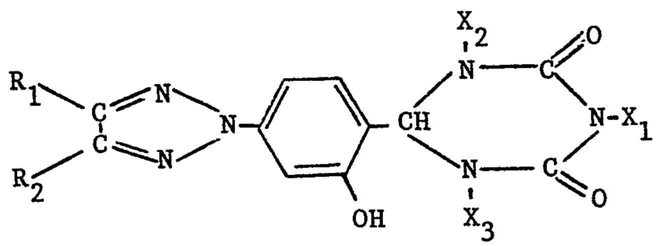

(I)

worin

$R_1$ und $R_2$ $C_1$-$C_4$-Alkyl oder gegebenenfalls durch Halogen, $C_1$-$C_4$-Alkyl oder $C_1$-$C_4$-Alkoxy substituiertes Phenyl

$X_1$, $X_2$ und $X_3$ $C_1$-$C_4$-Alkyl oder Phenyl-$C_1$-$C_4$-Alkyl, wobei der Phenylrest gegebenenfalls durch Halogen, $C_1$-$C_4$-Alkyl oder $C_1$-$C_4$-Alkoxy substituiert ist, bedeuten.

2. Triazolylphenyltriazine gemäß Anspruch 1, dadurch gekennzeichnet, daß $R_1$ und $R_2$ für Methyl, Ethyl oder Phenyl und $X_1$, $X_2$ und $X_3$ für Methyl stehen.

3. Verfahren zur Herstellung von Triazolylphenyltriazinen gemäß Anspruch 1, dadurch gekennzeichnet, daß man Triazolylphenole der Formel

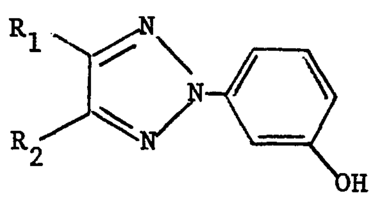

(II)

worin

$R_1$ und $R_2$ die in Anspruch 1 genannte Bedeutung haben, mit Verbindungen der Formel

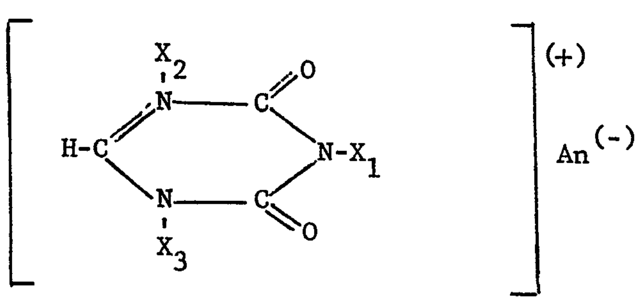

(III)

worin

$X_1$, $X_2$, $X_3$ die in Anspruch 1 genannte Bedeutung haben und

$An^{(-)}$ für ein Anion steht, in Gegenwart eines Säureakzeptor umsetzt.P

4. Verfahren gemäß Anspruch 3, dadurch gekennzeichnet, daß man die Umsetzung bei 80-140 °C, vorzugsweise 90-110 °C, durchführt.

5. Verfahren zur Herstellung von optischen Aufhellern der 7-Triazolylcumarin-Reihe, dadurch gekennzeichnet, daß man die Verbindungen gemäß Anspruch 1 durch alkalische Hydrolyse in die entsprechenden Salicylaldehyde überführt und diese mit (Het)-Arylessigsäuren in an sich bekannter PWeise kondensiert.

**Claims**

1. Triazolylphenyltriazines of the formula

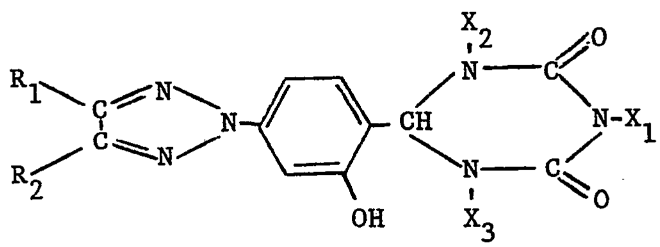

(I)

wherein

$R_1$ and $R_2$ denote $C_1$-$C_4$-alkyl or phenyl optionally substituted by halogen, $C_1$-$C_4$-alkyl or $C_1$-$C_4$-alkoxy $X_1$, $X_2$ and $X_3$ denote $C_1$-$C_4$-alkyl or phenyl-$C_1$-$C_4$-alkyl, the phenyl radical optionally being substituted by halogen, $C_1$-$C_4$-alkyl or $C_1$-$C_4$-alkoxy.

2. Triazolylphenyltriazines according to Claim 1, characterised in that $R_1$ and $R_2$ represent methyl, ethyl or phenyl and $X_1$, $X_2$ and $X_3$ represent methyl.

3. Process for the preparation of triazolylphenyltriazines according to Claim 1, characterised in that triazolylphenols of the formula

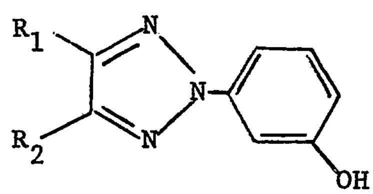

(II)

wherein

$R_1$ and $R_2$ have the meaning mentioned in Claim 1, are reacted with compounds of the formula

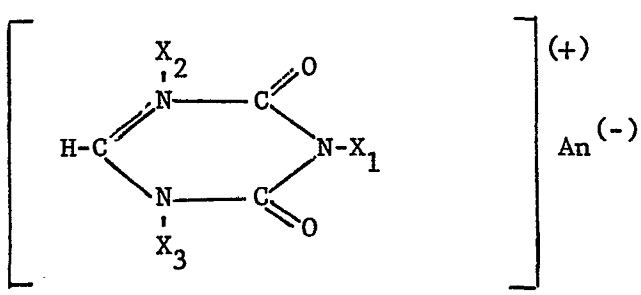

(III)

wherein

$X_1$, $X_2$ and $X_3$ have the meaning mentioned in Claim 1 and

$An^{(-)}$ represents an anion,

in the presence of an acid acceptor.

4. Process according to Claim 3, characterised in that the reaction is carried out at 80-140 °C, preferably 90-110 °C.

5. Process for the preparation of optical brighteners of the 7-triazolylcoumarin series, characterised in that the compounds according to Claim 1 are converted to the corresponding salicylaldehydes by

alkaline hydrolysis and the salicylaldehydes are condensed with arylacetic acids or hetarylacetic acids in a manner which is in itself known.

**Revendications**

1. Triazolylphényltriazines caractérisées en ce qu'elles répondent à la formule

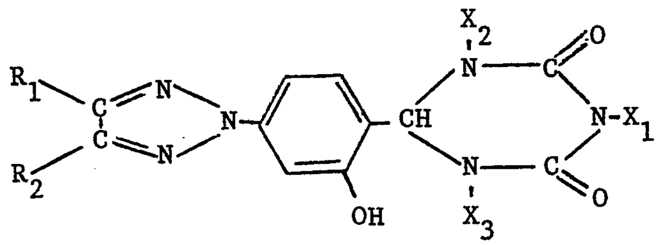

(I)

dans laquelle

$R_1$ et $R_2$ représentent un groupe alkyle en $C_1$-$C_4$ ou un groupe phényle éventuellement substitué par un halogène ou un groupe alkyle en $C_1$-$C_4$ ou alcoxy en $C_1$-$C_4$.

$X_1$, $X_2$ et $X_3$ représentent un groupe alkyle en $C_1$-$C_4$ ou phényl-alkyle en $C_1$-$C_4$, le reste phényle étant éventuellement substitué par un halogène ou par un groupe alkyle en $C_1$-$C_4$ ou alcoxy en $C_1$-$C_4$.

2. Triazolylphényltriazines selon la revendication 1, caractérisées en ce que $R_1$ et $R_2$ représentent un groupe méthyle, éthyle ou phényle et $X_1$, $X_2$ et $X_3$ représentent un groupe méthyle.

3. Procédé pour la préparation de triazolylphényltriazines selon la revendication 1, caractérisé en ce que l'on fait réagir des triazolylphénols de formule générale

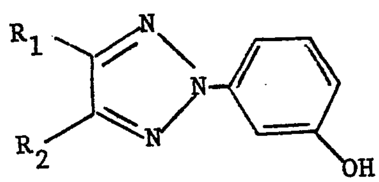

(II)

dans laquelle

$R_1$ et $R_2$ ont la signification indiquée à la revendication 1, avec des composés de formule

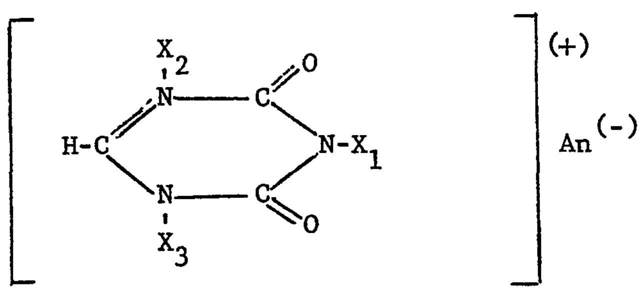

(III)

dans laquelle

$X_1$, $X_2$ et $X_3$ ont la signification indiquée à la revendication 1 et $An^{(-)}$ représente un anion, en présence d'un accepteur d'acide.

4. Procédé selon la revendication 3, caractérisé, en ce que l'on effectue la réaction à 80-140 °C, de préférence 90-110 °C.

5. Procédé pour la préparation d'éclaircissants optiques de la série de la 7-triazolylcoumarine, caractérisé en ce que l'on transforme les composés selon la revendication 1 en aldéhydes salicyliques correspondants par hydrolyse alcaline et l'on condense ceux-ci de manière connue avec des acides (hétéro)arylacétiques.